# EUROPÄISCHE PATENTSCHRIFT

(11) **EP 0 779 286 B1**
(45) Veröffentlichungstag und Bekanntmachung des Hinweises auf die Patenterteilung: **27.03.2002**
(21) Anmeldenummer: 96119504.7
(22) Anmeldetag: 05.12.1996
(51) Int. Cl.: C07D 307/62

(54) **Herstellung von Ascorbinsäure**
Preparation of ascorbic acid
Préparation de l'acide ascorbique

(30) Priorität: 14.12.1995 CH 354095
(43) Veröffentlichungstag der Anmeldung: 18.06.1997
(73) Patentinhaber: F. HOFFMANN-LA ROCHE AG, 4070 Basel (CH)
(72) Erfinder: Veits, Joachim, 79618 Rheinfelden (DE)
(74) Vertreter: Kellenberger, Marcus, Dr.

(56) Entgegenhaltungen:
- EP-A- 0 040 709
- WO-A-92/11080
- FR-A- 2 291 291
- GB-A- 871 500
- GB-A- 2 179 353
- US-A- 2 829 095
- PATENT ABSTRACTS OF JAPAN vol. 012, no. 306 (C-522), 19.August 1988 & JP 63 077890 A (NIPPON SAAFUAKUTANTO KOGYO KK), 8.April 1988,
- DATABASE WPI Week 9437 Derwent Publications Ltd., London, GB; AN 94-299797 XP002027068 & JP 06 228 183 A (UNITIKA LTD.) , 16.August 1994

## Beschreibung

Die vorliegende Erfindung betrifft ein elektrophysikalisches Verfahren zur Herstellung von Ascorbinsäure (Vitamin C) aus Ascorbaten.

Bei den erfindungsgemässen Herstellungsverfahren wird das in Wasser gelöste Ascorbat, vorzugsweise Natriumascorbat, unter dem Einfluss eines elektrischen Feldes in die entsprechenden Kationen (im allgemeinen als "Gegenionen" bezeichnet), z.B. Natriumionen (Na⁺), und Ascorbationen (Asc⁻) aufgespalten, die in von ionenselektiven Membranen begrenzten Säure - bzw. Basenräumen (Salzräumen) räumlich getrennt gesammelt werden. Im Säureraum werden in einer Ionenreaktion die Ascorbationen mit Protonen (H⁺) zu Ascorbinsäure (HAsc) zusammengefügt, während im Basenraum die Gegenionen (M⁺) mit Hydroxidionen (OH⁻) ebenfalls zusammengefügt werden, was als zweites Produkt der Ionenreaktion die diesbezügliche Base (MOH) ergibt. Dann können die daraus resultierenden wässrigen Lösungen von Ascorbinsäure und Base (Lauge) aus der Apparatur, in der sich das elektrophysikalische Verfahren abspielt, separat abgezogen werden. Das zusammen mit dem Hauptprodukt Ascorbinsäure durch das erfindungsgemässe Verfahren gewonnene Nebenprodukt MOH stellt selber einen Wertstoff dar und kann anderweitig eingesetzt werden. Im Verfahren, das unter anderem je nach Art der eingesetzten ionenselektiven Membranen sowie der eingesetzten Elektroden verschiedenen Ausführungsformen aufweist, können die Protonen grundsätzlich auf zwei verschiedene Arten zur Verfügung gestellt werden:
a) Durch eine elektrisch erzwungene Wasserdissoziation an einer bipolaren Membran, wobei es sich um eine Elektrodialyse handelt, und
b) Aus einer an einer monopolaren oder bipolaren Elektrode stattfindenden Elektrolyse.

Somit betrifft die vorliegende Erfindung ein Verfahren zur Herstellung von Ascorbinsäure ausgehend von einem Ascorbat, vorzugsweise, dem Natriumascorbat, das durch gekennzeichnet ist, dass man eine Wässrige Ascorbatlösung mit einer Konzentration an Ascorbat von etwa 15 bis etwa 45 Gewicht/Gewicht% bezogen auf die Lösung unter dem Einfluss eines elektrischen Feldes mittels ionenselektiver Membranen in Ascorbationen und Kationen zerlegt und räumlich voneinander trennt, und durch gleichzeitige Erzeugung von Protonen und Hydroxidionen dann die Ascorbinsäure aus den freigesetzten Ascorbationen und Protonen sowie räumlich getrennt davon auch noch das entsprechende Hydroxid aus den Kationen und Hydroxidionen herstellt, wobei die Protonen durch eine elektrisch erzwungene Wasserdissoziation an einer bipdaren Membran (Elektrodialyse) oder durch eine an einer monopolaren Elektrode Stattfindende Elektrolyse zur Verfügung gestellt werden. Danach können die so hergestellte Ascorbinsäure sowie - falls erwünscht-das Hydroxid aus den jeweiligen wässrigen Lösung isoliert werden.

Wie oben erwähnt, kann das erfindungsgemässe Verfahren sowohl mit Hilfe der Elektrodialyse als auch mit Hilfe der Elektrolyse verwirklicht werden.

Sowohl die Elektrodialyse mit monopolaren und bipolaren Membranen als auch die Elektrolyse mit monopolaren und bipolaren Elektroden sind seit einiger Zeit bekannte Verfahren. Beispielsweise ist der Einsatz der Elektrodialyse mit monopolaren und/oder bipolaren Membranen zur Gewinnung organischer Säuren ausführlich in der PCT-Patentpublikation WO 92/11080 beschrieben. Abgehandelt sind allerdings in dieser Patentpublikation nur Salze von Carbonsäuren, insbesondere Alkaliformiate (Natriumformiat) und Alkaliacetate. Ferner ist eine Reinigung der Ascorbinsäure mittels Elektrodialyse in der Deutschen Offenlegungsschrift (DOS) 3621 781 beschrieben. In dieser DOS werden Salze aus der Ascorbinsäure entfernt, die durch Neutralisation im Falle einer sauren Umlagerung in der Ascorbinsäure anfallen, bzw. die nach Freisetzung mittels Säure im Falle einer alkalischen Umlagerung aus dem Natriumascorbat vorliegen. In beiden Fällen wird neben der Ascorbinsäure ein Salzstrom erhalten.

Im Gegensatz zu diesem Stand der Technik wird durch das erfindungsgemässe Verfahren nicht nur Ascorbinsäure, sondern räumlich getrennt davon auch die entsprechende Lauge, z.B. Natriumhydroxid gewonnen. Zudem fallen bei der Durchführung des erfindungsgemässen Verfahrens Salze nicht an.

Bislang wird die Umsetzung von Natriumascorbat zu Ascorbinsäure im industriellen Massstab mit Hilfe von Ionenaustauscherharzen durchgeführt. Hierbei werden Natriumionen gegen die auf dem Austauscherharz befindlichen Protonen ausgetauscht, und auf diese Weise die Ascorbinsäure aus ihrem Salz freigesetzt. Ist die Kapazität des Harzes erschöpft, so muss dieses mit Säure regeneriert werden, wobei die Säure im Überschuss (kein stöchiometrischer Vorgang) eingesetzt werden muss. Hierbei fällt als Abfallprodukt eine saure Salzlösung an, die nachträglich neutralisiert und entsorgt werden muss, woraus sich ein zusätzlicher Aufwand und Kosten ergeben.

Die Vorteile der hier beschriebenen Erfindung liegen darin, dass die Umsetzung von Ascorbaten, vorzugsweise Natriumascorbat, zu Ascorbinsäure ohne den Einsatz zusätzlicher Chemikalien erfolgt. Weiterhin erhält man Lauge als Nebenprodukt, welche anderweitig als Wertstoff eingesetzt werden kann.

Die im erfindungsgemässen Verfahren beinhaltete räumliche Trennung der Kationen [beispielsweise Natriumionen (Na⁺)] und Ascorbationen (Asc⁻) voneinander und gleichzeitig Ausführungsformen dieses Verfahrens sind in den nachfolgenden Abbildungen dargestellt, in denen A eine (monopolare) Anionenaustauschermembran, B eine bipolare Membran und K eine Kationenaustauschermembran bedeuten:

### Abb. 1:

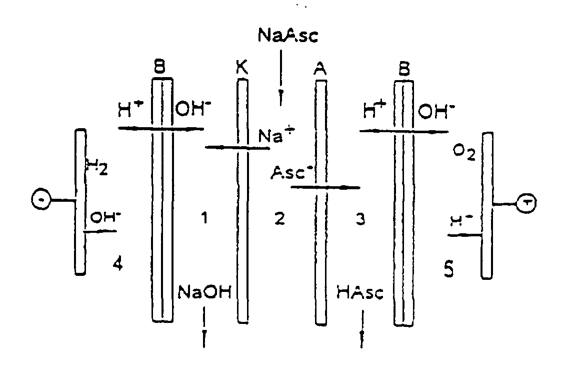

Legende: Herstellung von Ascorbinsäure aus Natriumascorbat mit Hilfe der Elektrodialyse mit bipolaren Membranen im **Dreikammersystem.**

### Abb.2:

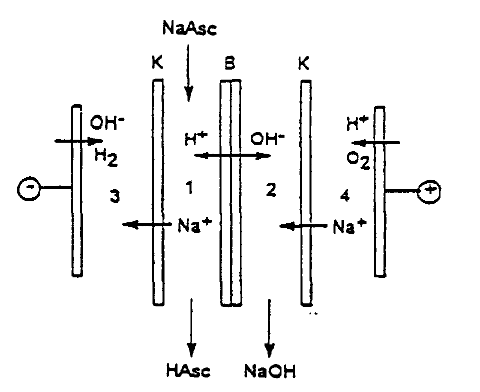

Legende:Herstellung von Ascorbinsäure aus Natriumascorbat mit Hilfe der Elektrodialyse mit bipolaren Membranen im **Zweikammersystem.**

### Abb.3:

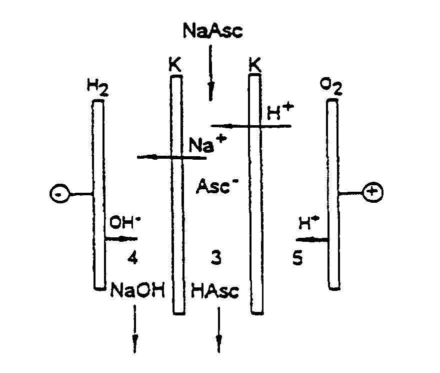

Legende:Herstellung von Ascorbinsäure aus Natriumascorbat mit Hilfe der Elektrolyse mit monopolaren Elektroden im **Dreikammersystem.**

### Abb. 4:

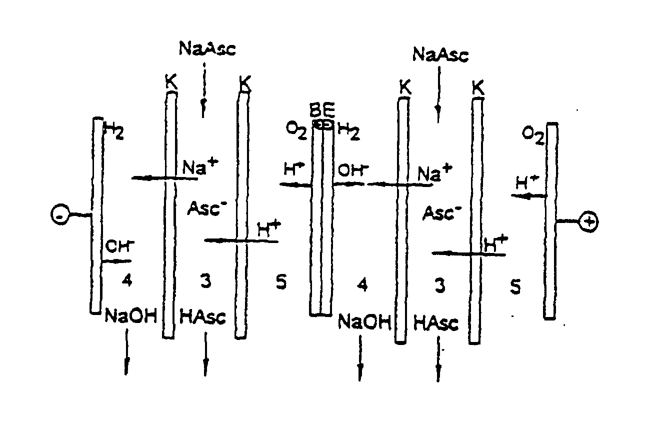

Legende:Herstellung von Ascorbinsäure aus Natriumascorbat mit Hilfe der Elektrolyse mit monopolaren Membranen und bipolaren Elektroden (BE) im **Dreikammersystem.**

### Abb. 5:

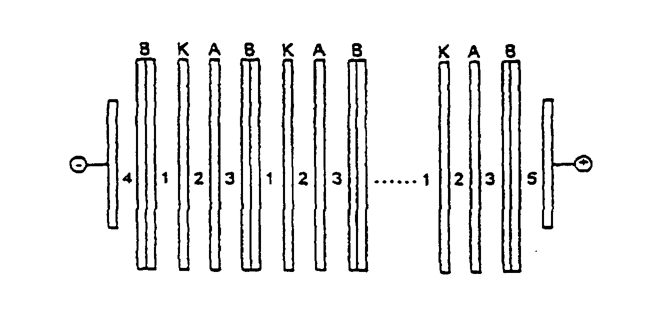

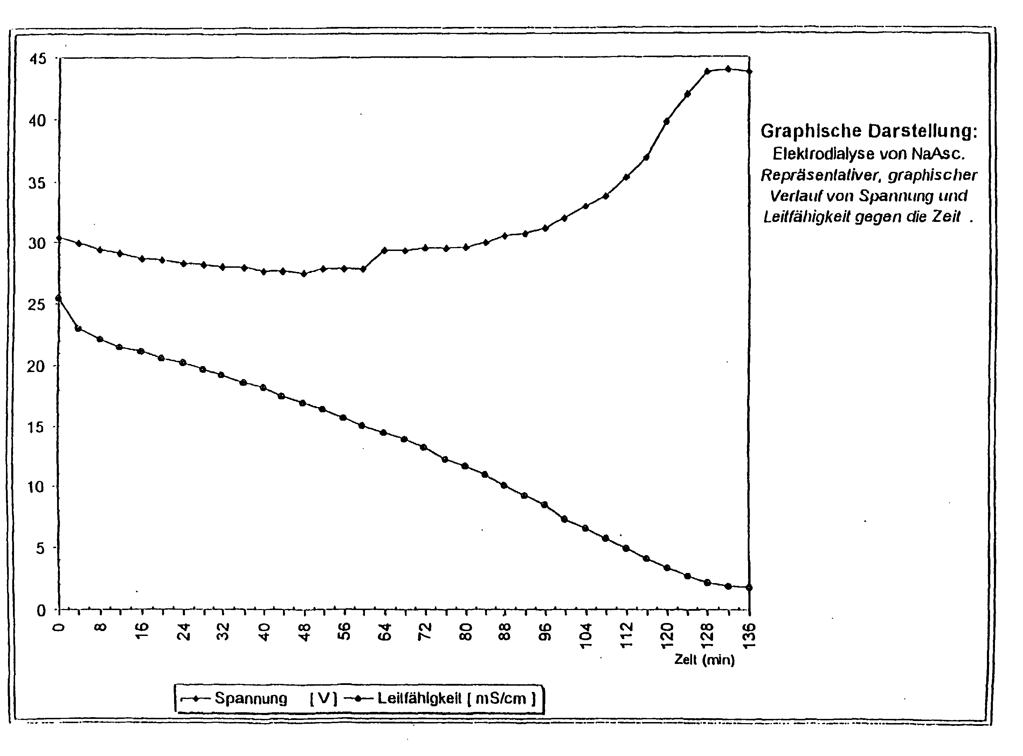

Legende:Anordnung mehrerer Dreikammerpakete für den beschriebenen Fall a), Abb. 1.

### Abb.6:

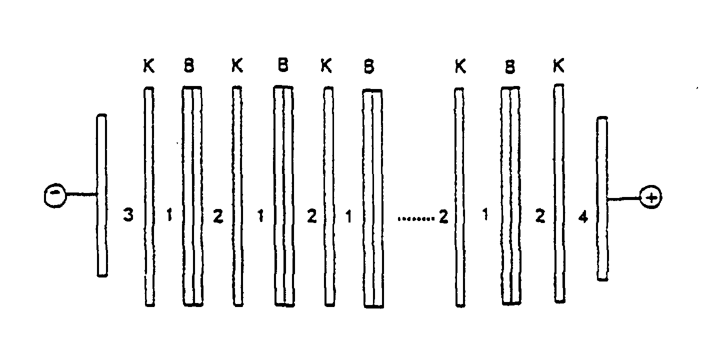

Legende:Anordnung mehrerer Zweikammerpakete für den Fall a), Abb. 2.

### Abb. 7:

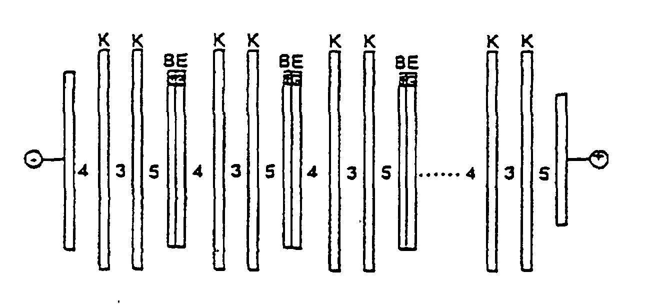

Legende:Anordnung mehrerer Dreikammerpakete für den Fall b), Abb. 4.

Die Elektrodialyse mit bipolaren Membranen kann sowohl in einem Dreikammersystem (Abb. 1) als auch in einem Zweikammersystem erfolgen (Abb. 2).

Im Fall der Erzeugung der Protonen an einer Elektrode gelangt man zu einem Dreikammersystem (Abb. 3, Abb. 4).

In den Fällen Abb. 1, 2 und 4 kann eine Vielzahl von Dreikammer- bzw. Zweikammerpaketen zwischen einem Elektrodenpaar angeordnet werden, wie in den Abb. 5, 6 und 7 dargestellt.

Die Erfindung wird nachstehend nun im einzelnen anhand der obigen Abbildungen 1 bis 7 erläutert.

### Abb. 1: Herstellung von Ascorbinsäure aus Natriumascorbat mit Hilfe der Elektrodialyse mit bipolaren Membranen im Dreikammersystem.

Natriumionen und Ascorbationen werden unter dem Einfluss eines elektrischen Feldes aus der von einer Natriumascorbatlösung durchströmten Kammer 2 entfernt und mit Hilfe ionenselektiver Membranen örtlich voneinander getrennt. Die Natriumionen wandern hierbei über eine Kationenaustauschermembran in Richtung Kathode und gelangen somit in den Basenkreislauf 1, wo sie mit den von einer bipolaren Membran produzierten Hydroxidionen zu Natronlauge reagieren. Die Ascorbationen wandern über eine Anionenaustauschermembran in Richtung Anode und gelangen somit in den Säurekreislauf 3, wo sie mit den von einer bipolaren Membran produzierten Protonen zu Ascorbinsäure reagieren. Die Elektrodenspülungen (Kammern 4 und 5) werden von einer Salzlösung durchströmt.

Die Anordnung einer beliebigen Anzahl von Dreikammerpaketen (Base, Natriumascorbat, Ascorbinsäure) zwischen einem Elektrodenpaar kann gemäss Abb. 5 stattfinden.

### Abb. 2: Herstellung von Ascorbinsäure aus Natriumascorbat mit Hilfe der Elektrodialyse mit bipolaren Membranen im Zweikammersystem.

Natriumionen werden unter dem Einfluss eines elektrischen Feldes aus der von einer Natriumascorbatlösung durchströmten Kammer 1 entfernt. Die Elektroneutralität in der Kammer 1 wird dadurch aufrechterhalten, dass jedes Natriumion von einem von der bipolaren Membran produzierten Proton ersetzt bzw. verdrängt wird.

Die Natriumionen wandern hierbei über eine Kationenaustauschermembran in Richtung Kathode und gelangen somit in den Basenkreislauf 2, wo sie mit den von einer bipolaren Membran produzierten Hydroxidionen zu Natronlauge reagieren. Die Ascorbationen reagieren im Säurekreislauf 1 mit den von der bipolaren Membran produzierten Protonen zu Ascorbinsäure. Die Eletrodenspülungen (Kammer 3 und 4) werden von einer Natriumhydroxid - bzw. Salzlösung - durchströmt.

Die Anordnung einer beliebigen Anzahl von Zweikammerpaketen (Base, Ascorbinsäure) zwischen einem Elektrodenpaar kann gemäss Abb. 6 stattfinden.

### Abb. 3: Herstellung von Ascorbinsäure aus Natriumascorbat mit Hilfe der Elektrolyse mit monopolaren Eletroden im Dreikammersystem.

Natriumionen werden unter dem Einfluss eines elektrischen Feldes aus der von einer Natriumascorbatlösung durchströmten Kammer 3 entfernt. Die Eletroneutralität in der Kammer 3 wird dadurch aufrechterhalten, dass jedes Natriumion von einem an der Anode im Kreislauf 5 produzierten Proton ersetzt bzw. verdrängt wird. Die Natriumionen gelangen hierbei unter dem Einfluss des elektrischen Feldes über eine Kationenaustauschermembran in Richtung Kathode und gelangen somit in den Basenkreislauf 4, wo sie mit den an der Kathode produzierten Hydroxidionen zu Natronlauge reagieren. Die Ascorbationen reagieren im Säurekreislauf 3 mit den an der Anode produzierten Protonen zu Ascorbinsäure. Die Kathodenspülung (Kammer 4) wird von der produzierten Base, die Anodenspülung (Kammer 5) von einer Säure (z.B. Schwefelsäure) durchströmt.

### Abb. 4: Herstellung von Ascorbinsäure aus Natriumascorbat mit Hilfe der Elektrolyse mit monopolaren Membranen und bipolaren Elektroden im Dreikammersystem

Natriumionen werden unter dem Einfluss eines elektrischen Feldes aus der von einer Natriumascorbatlösung durchströmten Kammer 3 entfernt. Die Elektroneutralität in der Kammer 3 wird dadurch aufrechterhalten, dass jedes Natriumion von einem an der Anode der bipolaren Elektrode (BE) oder monopolaren Elektrode im Kreislauf 5 produzierten Proton ersetzt bzw. verdrängt wird. Die produzierten Protonen gelangen hierbei unter dem Einfluss des elektrischen Feldes über eine Kationenaustauschermembran in die Säurekammer 3. Die Natriumionen wandern über eine Kationenaustauschermembran in Richtung Kathode und gelangen somit in den Basenkreislauf 4, wo sie mit den an der Kathode der bipolaren Elektrode (BE) oder monopolaren Elektrode produzierten Hydroxidionen zu Natronlauge reagieren. Die Ascorbationen reagieren im Säurekreislauf 3 mit den an der Anode produzierten Protonen zu Ascorbinsäure. Die Kathodenspülung (Kammer 4) wird von der produzierten Base, die Anodenspülung (Kammer 5) von einer Säure (z.B. Schwefelsäure) durchströmt.

Die Anordnung einer beliebigen Anzahl von Dreikammerpaketen (Base, Ascorbinsäure, Säure) zwischen einem Elektrodenpaar kann gemäss Abb. 7 stattfinden.

Im Rahmen der vorliegenden Erfindung ist unter dem Ausdruck "Ascorbat" - falls nicht spezifisch auf Natriumascorbat eingeschränkt-insbesondere ein Alkalimetall (M⁺)- oder ein Tertiärammonium (HNR₃⁺)- oder Quaternärammonium (NR₄⁺)-Ascorbat zu verstehen, wobei M⁺ insbesondere für das Lithium-, Natrium- oder Kaliumion und jedes R des Ions HNR₃⁺ oder NR₄⁺ insbesondere für eine geradkettige oder verzweigte Alkylgruppe, insbesondere eine Niederalkylgruppe, z.B. C₁₋₄-Alkylgruppe, vorzugsweise Methyl, steht. Vorzugsweise verwendet man als Ascorbat das Natriumascorbat.

Die im erfindungsgemässen Verfahren verwendete wässrige Ascorbatlösung weist eine Konzentration auf, die im allgemeinen im Bereich von etwa 15 bis etwa 45 Gewicht/Gewicht -% (G/G-%) bezogen auf die Lösung, vorzugsweise im Bereich von etwa 25 bis etwa 35 G/G-%, liegt. Auf jeden Fall muss unter Berücksichtigung der Art des Kations sowie der weiteren Verfahrensbedingungen, z.B. der Temperatur, die Konzentration so ausgewählt werden, dass das Ascorbat möglichst nicht aus der Lösung auskristallisiert.

Als im erfindungsgemässen Verfahren verwendete ionenselektive Membran eignet sich sowohl eine Anionen- oder Kationenaustauschermembran (monopolare Membran) als auch eine bipolare Membran. Bei den Anionenaustauschermembranen ("A" in den diesbezüglichen Abbildungen) handelt es sich im allgemeinen um stark, mild oder schwach basische Membranen, welche selektiv und für einwertige Anionen, nicht aber für einwertige Kationen, durchlässig sind. Beispiele solcher Membranen sind die Selemion® ASV-, AMV-, AAV- und ASV-Membran der Asahi Glass K.K., Japan, die Neosepta ACS-Membran der Tokuyama Soda K.K., ebenfalls Japan, sowie welche der Ionics Inc, Watertown, Mass., USA, insbesondere die Ionics 204-UZL-386-Membran dieser Firma. Die Kationenaustauschermembranen ("K" in den diesbezüglichen Abbildungen) sind hingegen mild oder stark saure Membranen, die beispielsweise Phosphorsäure- bzw. Sulfonsäuregruppen enthalten, permselektiv sind und bei dem angewendeten pH-Wert einen niedrigen elektrischen Widerstand aufweisen. Zudem lassen sie einwertige Kationen, aber keine einwertigen Anionen, durch. Diesbezügliche Beispiele sind die CMX- und die CMB-Membran der Tokuyama Soda K.K., die CMV-Membran der Asahi Glass K.K., die Nafion® 110-, 324-, 350- und 450-Membran der DuPont de Nemours, USA sowie die in der US-Patentschrift 4.738.764 (Chlanda et al.) beschriebenen Kationenaustauschermembranen. Die bipolaren Membranen ("B" in den diesbezüglichen Abbildungen) weisen eine Kation(+)- und eine Anion(-)-Schicht auf, wobei die erste für einwertige Kationen und die letztere für einwertige Anionen durchlässig ist. Zudem lässt die Kation-Schicht keine Anionen, und die Anion-Schicht keine Kationen, durch. Beispiele bipolarer Membranen sind die WSI-Membran der WSI Technologies Inc., St. Louis, Mo, USA, die FBI-Membran der FuMa-Tech, Deutschland, sowie die in den US-Patentschriften 2.829.095, 4.024.043, 4.082.835 und 4.116.889 beschriebenen bipolaren Membranen.

Die ideale Stromdichte bzw elektrische Spannung, bei der das erfindungsgemässe Verfahren durchgeführt wird, hängt jeweils von vielen weiteren Parametern ab, z.B. der Konzentration der wässrigen Ascorbatlösung, der Art und Anzahl der verwendeten Membranen und überhaupt deren Anordnung und Abmessungen (u.a. Kammerdicke, d.h. die Entfernung der Membranen voneinander) sowie der Temperatur, bei der das Verfahren durchgeführt wird, so dass sich die Grenzwerte nicht leicht-wenn überhaupt - bestimmen lassen. Infolge bisheriger Untersuchungen des erfindungsgemässen Verfahrens lässt sich allerdings erkennen, dass die Stromdichte geeigneterweise im Bereich von etwa 50 bis etwa 200 mA/cm² liegt. In der Praxis erweist es sich als nützlich, unter Messung der Produktionsrate der Ascorbinsäure die Stromdichte zu variieren, bis sich ein optimaler Verfahrensverlauf eingestellt hat.

Da Ascorbinsäure ausgeprägt hitzeempfindlich ist und schon bei nicht allzuviel erhöhter Temperatur, etwa ab 40°C, zu zersetzen anfängt, wird das erfindungsgemässe Verfahren zweckmässigerweise bei Temperaturen unterhalb von etwa 40°C, allerdings ebenfalls auch aus wirtschaftlichen Gründen oberhalb von etwa 10° C durchgeführt. Vorzugsweise wird innerhalb des Bereiches von etwa 15° C bis etwa 25°C verfahren.

Darüber hinaus weist das erfindungsgemässe Verfahren in bezug auf die Art und Anordnung der verwendeten Membranen und Elektroden und beispielsweise unter Verwendung des bevorzugten Ascorbats Natriumascorbat mehrere bevorzugte Ausführungsformen auf, und zwar:
(i) Das Verfahren wird so durchgeführt, dass die Protonen und Hydroxidionen durch eine elektrisch erzwungene Wasserdissoziation an einer bipolaren Membran freigesetzt werden und anschliessend räumlich voneinander getrennt mit den Ascorbationen und den Natriumionen zu Ascorbinsäure bzw. Natriumhydroxid reagieren. Bei dieser Ausführungsform handelt es sich um eine Elektrodialyse, wie diese beispielsweise durch die vorher angegebenen Abbildungen 1 und 2 (noch spezifischer, Abbildung 5 bzw. 6) und die diesbezüglichen Erläuterungen veranschaulicht werden kann. Vorzugsweise wird diese Ausführungsform des erfindungsgemässen Verfahrens so durchgeführt, dass mehrere Dreikammerpakete bestehend jeweils aus einer Basen-, einer Natriumascorbat- und einer Ascorbinsäurekammer hintereinander zwischen einem Elektrodenpaar angeordnet sind, wie dies beispielsweise durch die Abbildung 5 veranschaulicht werden kann.
(ii) Das Verfahren wird so durchgeführt, dass die Natriumionen des Natriumascorbats in der wässrigen Lösung durch die infolge einer elektrisch erzwungenen Wasserdissoziation an einer bipolaren Membran freigesetzten Protonen ersetzt bzw. verdrängt werden und unter dem Einfluss des elektrischen Feldes über eine Kationenaustauschermembran in den benachbarten Kreislauf transportiert werden, wo sie mit den an der bipolaren Membran erzeugten Hydroxidionen zu Natriumhydroxid reagieren. Bei dieser Ausführungsform handelt es sich ebenfalls um eine Elektrodialyse, wie dies beispielsweise durch die Abbildung 2 und die diesbezügliche Erläuterung veranschaulicht werden kann. Vorzugsweise wird diese Ausführungsform des erfindungsgemässen Verfahrens so durchgeführt, dass mehrere Zweikammerpakete bestehend jeweils aus einer Basenkammer und einer Ascorbinsäurekammer hintereinander zwischen einem Elektrodenpaar angeordnet sind, wie dies beispielsweise durch die Abbildung 6 veranschaulicht werden kann.
(iii)Das Verfahren wird so durchgeführt, dass die Natriumionen des Natriumascorbats in der wässrigen Lösung durch die an einer bipolaren Elektrode freigesetzten Protonen ersetzt bzw. verdrängt werden und in einer durch eine Kationenaustauschermembran getrennten Kammer mit den an der bipolaren Elektrode freigesetzten Hydroxidionen zu Natriumhydroxid reagieren. Bei dieser Ausführungsform handelt es sich um eine Elektrolyse, wie diese beispielsweise durch die vorher angegebene Abbildung 4 und die diesbezügliche Erläuterung veranschaulicht werden kann. Vorzugsweise wird diese Ausführungsform des erfindungsgemässen Verfahrens so durchgeführt, dass mehrere Dreikammerpakete bestehend jeweils aus einer Basen-, einer Ascorbinsäure- und einer Säurekammer hintereinander zwischen einem monopolaren Elektrodenpaar angeordnet sind, wie dies beispielsweise durch die Abbildung 7 veranschaulicht werden kann.

Die vorliegende Erfindung wird durch das nachfolgende Beispiel veranschaulicht:

### Beispiel

### Durchführung der Elektrodialyse:

Ca. 300 g Natriumascorbat wurden in entionisiertem Wasser gelöst, so dass letztlich eine ca. 30 G/G-%ige Natriumascorbatlösung resultierte. Diese wurde in den Kreislauf 1 (siehe nachstehende Abbildung 8) transferiert und bei einer Stromstärke von 5 A umgepumpt (ca. 30 l / h). Die Umsetzung wurde solange durchgeführt, bis die Leitfähigkeit im Produktkreislauf auf etwa 1,7 bis 2,2 mS / cm abgefallen war. Der Elektrodenspülkreislauf 2 wurde mit einer 0,1 molaren Natriumhydroxidlösung betrieben (ca. 40 l / h). Der Natriumhydroxid-Kreislauf 3 wurde mit einer Leitfähigkeit von 20 mS / cm gestartet (ca. 20 l / h). Die Kreisläufe 1 bis 3 wurden durch einen externen Kühlkreislauf gekühlt.

Insgesamt wurden auf die beschriebene Weise ca. 200 Elektrodialysen mit rohem Natriumascorbat ausgeführt, wobei die eingesetzte Natriumascorbatmenge im Bereich von ca. 270 bis 350 g pro Ansatz lag.

Nach Erreichen einer Endleitfähigkeit von ca. 2 mS/cm wird die Elektrodialyse abgebrochen und die wässrige Ascorbinsäurelösung (ca. 22-25 G/G-%ig) aus dem Kreislauf 1 (Behälter) entnommen und weiter aufgearbeitet.

Nach Entfernung restlicher Natriumionen (ca. 500 ppm) und üblicher Aufarbeitung wurde stets den Spezifikationen entsprechende Ascorbinsäure isoliert.

### Angaben zum Elektrodialysemodul:

Die Elektrodialyse erfolgte mit einem kommerziellen Modul (Zweikammersystem) der Firma GOEMA (Vaihingen / Enz, Deutschland). Die Kammerdicke belief sich auf 2 mm und das Modul war mit 4 Zellpaaren mit einer Membranfläche von je 36 cm² (= 144 cm² Gesamtfläche) ausgerüstet. An Membranen wurde als Kationenaustauschermembran eine CMX-Membran (Tokuyama Soda Co. Ltd. Japan), als bipolare Membran eine WSI- bzw. eine FBI-Membran (WSI Technologies Inc., St. Louis, Mo, USA bzw. FuMa-Tech, Deutschland) eingesetzt. Als Elektroden wurden Mischmetalloxid-Elektroden eingesetzt.

### Weitere Angaben:

| | |
|---|---|
| Stromstärke | 5,0 Ampere (konstant) |
| Spannung | 28 bis 44 Volt |
| Membranfläche | 144 cm² |
| Produktionsrate | 120 bis 128 g Asc / h |
| Wiederfindung | 98,5 bis 99,5 % (bez. Ascorbat) |
| Rest-Na (in Asc) | ca. 500 ppm (Durchschnittswert) |

Die Stromausbeuten lagen im Bereich von 92 bis 98 %, überwiegend bei 95 %.

Nachstehend abgebildet ist eine Zeichnung der Elektrodialyseapparatur, mit der die Umwandlung von Natriumascorbat zu Ascorbinsäure durchgeführt wurde.

### Abbildung 8: Elektrodialyseeinheit mit 4 Zellpaaren

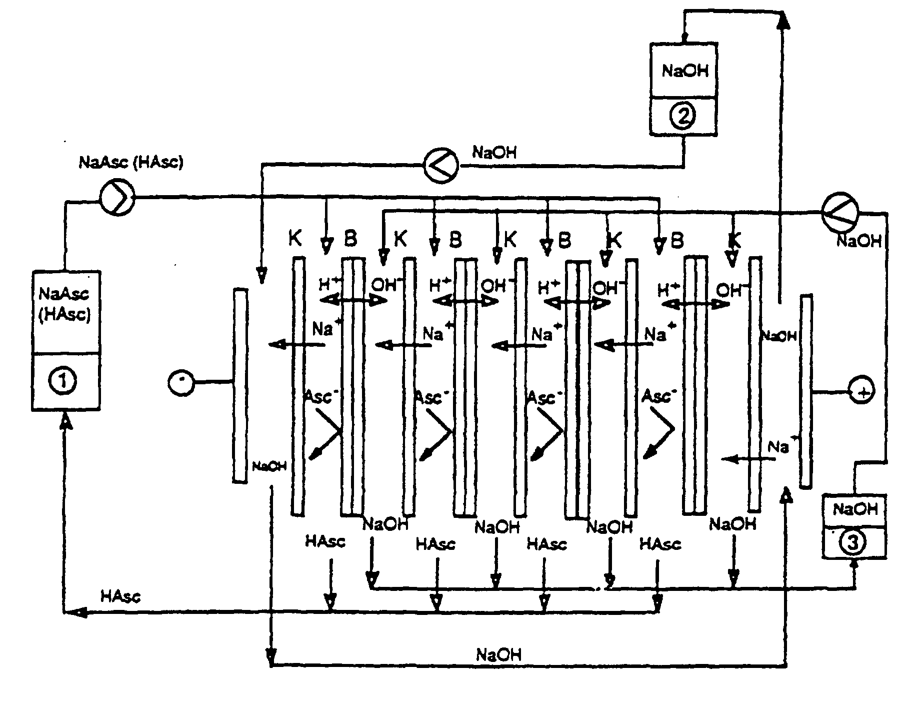

### Legende:

K: Kationenaustauschermembran
B: Bipolare Membran
Kreislauf 1: NaAsc (HAsc)-Kreislauf (Produktkreislauf)
Kreislauf 2: Elektrodenspülkreislauf (-0,1 m NaOH)
Kreislauf 3: NaOH-Kreislauf (Produktkreislauf)

Nachstehend eine tabellarische Übersicht mit Messwerten (Spannung, Leitfähigkeit) als Funktion der Zeit einer Elektrodialyse von Natriumascorbat:

| Zeit [Min] | Spannung [V] | Leitfähigkeit [mS/cm] |
|---|---|---|
| 0 | 30,5 | 25,6 |
| 4 | 30,0 | 23,0 |
| 8 | 29,5 | 22,1 |
| 12 | 29,2 | 21,5 |
| 16 | 28,8 | 21,1 |
| 20 | 28,7 | 20,6 |
| 24 | 28,4 | 20,2 |
| 28 | 28,3 | 19,7 |
| 32 | 28,1 | 19,2 |
| 36 | 28,0 | 18,6 |
| 40 | 27,8 | 18,2 |
| 44 | 27,8 | 17,5 |
| 48 | 27,6 | 17,0 |
| 52 | 27,9 | 16,4 |
| 56 | 27,9 | 15,7 |
| 60 | 27,9 | 15,1 |
| 64 | 29,4 | 14,4 |
| 68 | 29,4 | 13,9 |
| 72 | 29,6 | 13,2 |
| 76 | 29,6 | 12,2 |
| 80 | 29,7 | 11,6 |
| 84 | 30,0 | 10,9 |
| 88 | 30,6 | 10,0 |
| 92 | 30,8 | 9,2 |
| 96 | 31,2 | 8,4 |
| 100 | 32,0 | 7,3 |
| 104 | 33,0 | 6,5 |
| 108 | 33,8 | 5,7 |
| 112 | 35,4 | 4,9 |
| 116 | 37,0 | 4,1 |
| 120 | 39,8 | 3,4 |
| 124 | 42,0 | 2,7 |
| 128 | 43,8 | 2,2 |
| 132 | 44,0 | 1,9 |
| 136 | 43,8 | 1,8 |

Nachstehend ein repräsentativer, graphischer Verlauf einer Elektrodialyse.

Aufgetragen wurden die Spannung und Leitfähigkeit gegen die Zeit:

## Patentansprüche

1. Verfahren zur Herstellung von Ascorbinsäure ausgehend von einem Ascorbat, **dadurch gekennzeichnet, dass** man eine Wässrige Ascorbatlösung mit einer Konzentration an Ascorbat von etwa 15 bis etwa 45 Gewicht/Gewicht% bezogen auf die Lösung unter dem Einfluss eines elektrischen Feldes mittels ionenselektiver Membranen in Ascorbationen und Kationen zerlegt und räumlich voneinander trennt, und durch gleichzeitige Erzeugung von Protonen und Hydroxidionen dann die Ascorbinsäure aus den freigesetzten Ascorbationen und Protonen sowie räumlich getrennt davon auch noch das entsprechende Hydroxid aus den Kationen und Hydroxidionen herstellt, wobei die Protonen durch eine elektrisch erzwungene Wasserdissoziation an einer bipdaren Membran (Elektrodialyse) oder durch eine an einer monopolaren oder bipolaren Elektrode Stattfindende Elektrolyse zur Verfügung gestellt werden.

2. Verfahren nach Anspruch 1, **dadurch gekennzeichnet. dass** als Ascorbat Natriumascorbat verwendet wird.

3. Verfahren nach Anspruch 1 oder 2, **dadurch gekennzeichnet, dass** die wässrige Ascorbatlösung eine Konzentration von etwa 25 bis etwa 35 Gewicht/Gewicht-% bezogen auf die Lösung aufweist.

4. Verfahren nach Anspruch 2 oder 3, **dadurch gekennzeichnet, dass** die Protonen und Hydroxidionen durch eine elektrisch erzwungene Wasserdissoziation an einer bipolaren Membran freigesetzt werden und anschliessend räumlich voneinander getrennt mit den Ascorbationen und den Natriumionen zu Ascorbinsäure bzw. Natriumhydroxid reagieren.

5. Verfahren nach Anspruch 4, **dadurch gekennzeichnet, dass** es so durchgeführt wird, dass mehrere Dreikammerpakete bestehend jeweils aus einer Basen-, einer Natriumascorbat- und einer Ascorbinsäurekammer hintereinander zwischen einem Elektrodenpaar angeordnet sind.

6. Verfahren nach Anspruch 2 oder 3, **dadurch gekennzeichnet, dass** die Natriumionen des Natriumascorbats in der wässrigen Lösung durch die infolge einer elektrisch erzwungenen Wasserdissoziation an einer bipolaren Membran freigesetzten Protonen ersetzt bzw. verdrängt werden und unter dem Einfluss des elektrischen Feldes über eine Kationenaustauschermembran in den benachbarten Kreislauf transportiert werden, wo sie mit den an der bipolaren Membran erzeugten Hydroxidionen zu Natriumhydroxid reagieren.

7. Verfahren nach Anspruch 6, **dadurch gekennzeichnet, dass** es so durchgeführt wird, dass mehrere Zweikammerpakete bestehend jeweils aus einer Basenkammer und einer Ascorbinsäurekammer hintereinander zwischen einem Elektrodenpaar angeordnet sind.

8. Verfahren nach Anspruch 2 oder 3, **dadurch gekennzeichnet, dass** die Natriumionen des Natriumascorbats in der wässrigen Lösung durch die an einer bipolaren Elektrode freigesetzten Protonen ersetzt bzw. verdrängt werden und in einer durch eine Kationenaustauschermembran getrennten Kammer mit den an der bipolaren Elektrode freigesetzten Hydroxidionen zu Natriumhydroxid reagieren

9. Verfahren nach Anspruch 8, **dadurch gekennzeichnet, dass** es so durchgeführt wird, dass mehrere Dreikammerpakete bestehend jeweils aus einer Basen-, einer Ascorbinsäure- und einer Säurekammer hintereinander zwischen einem monopolaren Elektrodenpaar angeordnet sind.

## Claims

1. A process for the preparation of ascorbic acid starting from an ascorbate, **characterized in that** an aqueous ascorbate solution with an ascorbate concentration of about 15 to about 45 weight/weight % relative to the solution is decomposed under the influence of an electric field by means of ion-selective membranes into ascorbate ions and cations and the latter are separated spatially from one another, and then, by simultaneous generation of protons and hydroxide ions, the ascorbic acid is prepared from the liberated protons and ascorbate ions and, spatially separated therefrom, the corresponding hydroxide is also prepared from the cations and hydroxide ions, in such a way that the protons are made available by an electrically induced water dissociation at a bipolar membrane (electrodialysis) or by electrolysis occurring at a monopolar or bipolar electrode.

2. The process according to Claim 1, **characterized in that** sodium ascorbate is used as the ascorbate.

3. The process according to Claim 1 or 2, **characterized in that** the aqueous ascorbate solution has a concentration of about 25 to about 35 weight/weight % relative to the solution.

4. The process according to Claim 2 or 3, **characterized in that** the protons and hydroxide ions are liberated by an electrically induced water dissociation at a bipolar membrane and then react with the ascorbate ions and the sodium ions to form ascorbic acid and sodium hydroxide, respectively, while spatially separated from one another.

5. The process according to Claim 4, **characterized in that** it is carried out in such a way that a plurality of three-chamber assemblies each comprising a base chamber, a sodium ascorbate chamber and an ascorbic acid chamber are arranged behind one another between an electrode pair.

6. The process according to Claim 2 or 3, **characterized in that** the sodium ions of the sodium ascorbate in the aqueous solution are replaced or displaced by the protons liberated as a consequence of an electrically induced water dissociation at a bipolar membrane and are transported under the influence of the electric field via a cation exchanger membrane into the adjacent circuit, where they react with the hydroxide ions produced at the bipolar membrane to form sodium hydroxide.

7. The process according to Claim 6, **characterized in that** it is carried out in such a way that a plurality of two-chamber assemblies each comprising a base chamber and an ascorbic acid chamber are arranged behind one another between an electrode pair.

8. The process according to Claim 2 or 3, **characterized in that** the sodium ions of the sodium ascorbate in the aqueous solution are replaced or displaced by the protons liberated at a bipolar electrode and react with the hydroxide ions liberated at the bipolar electrode to form sodium hydroxide in a chamber separated by a cation exchanger membrane.

9. The process according to Claim 8, **characterized in that** it is carried out in such a way that a plurality of three-chamber assemblies each comprising a base chamber, an ascorbic acid chamber and an acid chamber are arranged behind one another between a monopolar electrode pair.

## Revendications

1. Procédé de préparation d'acide ascorbique à partir d'un ascorbate, **caractérisé en ce qu'**on dissocie sous l'influence d'un champ électrique une solution aqueuse d'ascorbate, ayant une concentration en ascorbate d'environ 15 à environ 45% en poids par rapport à la solution, en ions ascorbate et en cations qui sont séparés au moyen de membranes séparatrices d'ions, et grâce à la production simultanée de protons et d'ions hydroxyde on produit l'acide ascorbique à partir des ions ascorbate et des protons et, separé de ceux ci, l'hydroxyde correspondant à partir des cations et des ions hydroxyde, les protons provenant de la dissociation de l'eau par un courant électrique au niveau d'une membrane bipolaire (électrodialyse) ou par une electrolyse ayant lieu au niveau d'une électrode monopolaire ou bipolaire.

2. Procédé selon la revendication 1, **caractérisé en ce qu'**on utilise en tant qu'ascorbate l'ascorbate de sodium.

3. Procédé selon la revendication 1 ou 2, **caractérisé en ce que** la solution aqueuse d'ascorbate a une concentration d'environ 25 à environ 35% en poids par rapport à la solution.

4. Procédé selon la revendication 2 ou 3, **caractérisé en ce que** les protons et les ions hydroxyde sont libérés par une dissociation de l'eau au niveau d'une membrane bipolaire par un courant électrique, puis, en étant séparés les uns des autres , ils réagissent avec les ions ascorbate et les ions sodium pour donner de l'acide ascorbique et de l'hydroxyde de sodium.

5. Procédé selon la revendication 4, **caractérisé en ce qu'**il est mis en oeuvre de telle sorte que plusieurs groupes de trois compartiments, constitués chacun d'un compartiment de base, d'un compartiment d'ascorbate de sodium et d'un compartiment d'acide ascorbique, sont disposés l'un derrière l'autre entre deux électrodes.

6. Procédé selon la revendication 2 ou 3, **caractérisé en ce que** les ions sodium de l'ascorbate de sodium dans la solution aqueuse sont remplacés ou déplacés par les protons qui sont libérés au niveau d'une membrane bipolaire en conséquence d'une dissociation de l'eau par un courant électrique, et, sous l'influence du champ électrique, sont transportés par l'intermédiaire d'une membrane échangeuse de cations dans le circuit voisin, où ils réagissent avec les ions hydroxyde, produits au niveau de la membrane bipolaire, pour donner de l'hydroxyde de sodium.

7. Procédé selon la revendication 6, **caractérisé en ce qu'**il est mis en oeuvre de telle sorte que plusieurs groupes de deux compartiments, constitués chacun d'un compartiment de base et d'un compartiment d'acide ascorbique, soient disposés l'un derrière l'autre entre deux électrodes.

8. Procédé selon la revendication 2 ou 3, **caractérisé en ce que** les ions sodium de l'ascorbate de sodium sont, dans la solution aqueuse, remplacés ou déplacés par les protons libérés au niveau d'une électrode bipolaire, et, dans une chambre séparée par une membrane échangeuse de cations, réagissent avec les ions hydroxyde libérés au niveau de l'électrode bipolaire, pour donner de l'hydroxyde de sodium.

9. Procédé selon la revendication 8, **caractérisé en ce qu'**il est mis en oeuvre par le fait que plusieurs groupes de trois compartiments, constitués chacun d'un compartiment de base, d'un compartiment d'acide ascorbique et d'un compartiment d'acide, sont disposés l'un derrière l'autre entre deux électrodes monopolaires.
